(19)

**Europäisches Patentamt**

**European Patent Office**

**Office européen des brevets**

(11) **EP 4 389 093 A1**

(12) **EUROPEAN PATENT APPLICATION**

(43) Date of publication:
**26.06.2024 Bulletin 2024/26**

(21) Application number: **23218904.3**

(22) Date of filing: **20.12.2023**

(51) International Patent Classification (IPC):
***A61F 13/49*** (2006.01)

(52) Cooperative Patent Classification (CPC):
**A61F 13/49012**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.12.2022 EP 22216567**

(71) Applicants:
 • **Ontex BV**
  **9255 Buggenhout (BE)**

 • **Ontex Group NV**
  **9320 Erembodegem (BE)**

(72) Inventors:
 • **Donatella, Paola Montepara**
  **66036 Orsogna (IT)**
 • **Strazzella, Daniela**
  **66023 Francavilla al Mare, Chieti (IT)**
 • **Paolini, Pierguido**
  **66026 Ortona (IT)**

(74) Representative: **Macchetta, Andrea**
 **Ontex BV**
 **Korte Keppestraat 21**
 **9320 Erembodegem-Aalst (BE)**

(54) **ABSORBENT ARTICLES WITH IMPROVED ELASTOMERIC LAMINATES**

(57) An absorbent article comprising an absorbent chassis comprising a topsheet, a backsheet and an absorbent core disposed between the topsheet and the backsheet. The absorbent article comprises a first elastomeric laminate forming at least a portion of a belt, a side panel, or an ear panel in a front waist region and a second elastomeric laminate forming at least a portion of a belt, a side panel, or an ear panel in a back waist region. The first elastomeric laminate comprises a first plurality of elastics disposed between a first substrate and a second substrate and having an Average-Dtex of less than 600, an Average-Strand-Spacing of more than 4 mm and an Average-Pre-Strain of from 50% to about 400%. The second elastomeric laminate comprises a second plurality of elastics disposed between a first substrate and a second substrate and having an Average-Dtex of less than 600, an Average-Strand-Spacing of more than 4 mm and an Average-Pre-Strain of from 50% to about 400%. The first and/or second plurality of elastics comprise from about 0,1% to about 99,9%wt of recycled polymers by total weight of polymers, preferably from about 10% to about 90%wt of recycled polymers by total weight of polymers.

FIG. 2

EP 4 389 093 A1

## Description

## TECHNICAL FIELD

The present disclosure relates to absorbent articles, more particularly, to disposable absorbent articles comprising improved elastomeric laminates. The present disclosure refers to elastomeric laminates comprising elastic strands with recycled polymers having a greater fineness.

## BACKGROUND

Elastomeric laminates of the prior art have a number of consumer negatives that vary based on the laminate structure. Traditional laminates comprising elastic strands of relatively high dtex and relatively large elastic strand spacing have the disadvantage that each individual elastic exerts high pressure on the skin leading to increased skin marking and reduced comfort.

Traditional stranded laminates typically comprise elastics spaced at least 4 mm apart primarily due to manufacturing limitations and handling of individual strands of elastics via separate material infeeds.

With regard to extruded strands and/or extruded scrim materials, they are similar to many elastomeric films in that they typically comprise thermoplastic materials that undergo significant stress relaxation over time and thus do not maintain the proper forces at the waist and legs to provide proper initial and sustained fit and gasketing over the entire wearing time.

In terms of elastic film laminates, these are significantly less breathable with very low or no air permeability. This greater occlusive effect results in greater hydration of the skin and thus reduced comfort and increased marking. Elastic film laminates tend to have a higher modulus versus elastic laminates comprising elastic strands. Therefore they are mor difficult to apply to a wearer, therefor requiring more sizes to cover a given fit range of wearers. It is also very difficult to create a force profile across the elastic film laminate or scrim based elastic laminates.

Elastic laminates comprising elastic strands have been disclosed comprising a greater number of elastic strands having a greater fineness and a closer strand spacing. However these elastic stranded laminates require a complete structural redesign of the elastomeric laminates used in the absorbent article. A specific and unique blend of properties is required which results in the necessity for a new process in order to deliver the large amount of low decitex elastics at the low spacing.

There has therefor been a long standing unmet consumer need to create a product that delivers very low pressure on the skin, high level of breathability, adequate force for sustained fit, low modulus, high extensibility and a smooth uniform texture without the requirement/need to develop a completely new process and avoiding high investment costs. In light of the continuous search for improvements in terms of sustainability goals, there is a need to further improve the efficiency of raw material usage within elastic stranded laminates. A further need is identified as being the necessity for an improved coordination between product characteristics and proper usage of sustainable packaging materials.

## SUMMARY

In a first aspect, the present invention relates to an absorbent article comprising an absorbent insert. The absorbent insert comprises a topsheet, a backsheet and an absorbent core disposed between the topsheet and the backsheet, wherein the absorbent insert comprises a front end edge and a back end edge. The absorbent article comprises a first elastomeric laminate forming at least a portion of a belt, a side panel, or an ear panel in a front waist region, wherein the first elastomeric laminate comprises a first plurality of elastics disposed between a first substrate layer and a second substrate layer and wherein the first plurality of elastics comprises an Average-Dtex of less than 600, an Average-Strand-Spacing of more than 4 mm and an Average-Pre-Strain of from 50% to about 400%. Furthermore, the absorbent article comprises a second elastomeric laminate forming at least a portion of a belt, a side panel, or an ear panel in a back waist region, wherein the second elastomeric laminate comprises a second plurality of elastics between a first substrate layer and a second substrate layer and wherein the second plurality of elastics comprises an Average-Dtex of less than 600, an Average-Strand-Spacing of more than 4 mm and an Average-Pre-Strain of from 50% to about 400%. The first plurality of elastics and/or the second plurality of elastics comprise from about 0,1% to about 99,9%wt of recycled polymers by total weight of polymers, preferably from about 10% to about 90%wt of recycled polymers by total weight of polymers.

In a second aspect, the present invention relates to a package comprising paper material including absorbent articles according to the first aspect.

In a third aspect, the present invention relates to an array of packages comprising two or more sizes comprising absorbent articles according to the first aspect.

## BRIEF DESCRIPTION OF THE FIGURES

FIG. 1 shows, in plan view, an exemplary embodiment of an absorbent chassis

FIG. 2 shows, in plan view, an exemplary embodiment according to the present invention in extended state

## DESCRIPTION

The present invention provides for improved elastomeric laminates which can be used as components in disposable absorbent articles for fit and gasketing at the waist, legs, crotch and sides of the wearer to generally provide the greatest level of extensibility, the most comfortable wearing conditions, improved leakage protection, better fit and efficient usage of raw materials. Surprisingly, it has been found that including recyclable polymers in elastic strands safeguards the performance characteristics when reducing the Dtex of these strands.

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

The expression "% by weight" (weight percent) otherwise referred to as "%wt", here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed invention. However, it is to be understood that the value to which the modifier "about" refers is itself also specifically disclosed.

The term "absorbent article" refers to a device that absorbs and contains liquid, and more specifically, refers to a device that is placed against or in proximity to the body of the wearer to absorb and contain the various wastes/exudates discharged from the body. Absorbent articles include but are not limited to diapers, adult incontinence briefs, training pants and diaper holders and liners.

"Array" means a display of packages comprising disposable absorbent articles of different article constructions (e.g., different elastomeric materials [compositionally and/or structurally] in the side panels, side flaps and/or belts flaps, different graphic elements, different product structures, fasteners or lack thereof, sizes, core capacities, etc.). The packages may have the same brand and/or sub-brand and/or the same trademark registration and/or having been manufactured by or for a common manufacturer and the packages may be available at a common point of sale (e.g. oriented in proximity to each other in a given area of a retail store). An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Huggies," and same sub-brand, for example, "Pull-Ups." A different product in the array may have the same brand "Huggies" and the sub-brand "Little Movers." The differences between the "Pull-Ups"product of the array and the "Little Movers" product in the array may include product form, application style, different fastening designs or other structural elements intended to address the differences in physiological or psychological development. Furthermore, the packaging is distinctly different in that "Pull-Ups" is packaged in a predominately blue or pink film bag and "Little Movers" is packaged in a predominately red film bag. Further regarding "Arrays," as another example an array may be formed by different products having different product forms manufactured by the same manufacturer, for example, "Kimberly-Clark", and bearing a common trademark registration for example, one product may have the brand name "Huggies," and sub-brand, for example, "Pull-Ups." A different product in the array may have a brand/sub-brand "Good Nites" and both are registered trademarks of The Kimberly-Clark Corporation and/or are manufactured by Kimberly-Clark. Arrays also often have the same trademarks, including trademarks of the brand, sub-brand, and/or features and/or benefits across the line-up. "On-line Array" means an "Array" distributed by a common on-line source.

The term "channel" as used herein, is a region or zone in an absorbent material layer that has a substantially lower basis weight (e.g., less than 50%, less than 70%, less than 90%) than the surrounding material in the material layer. The channel may be a region in a material layer that is substantially absorbent material-free (e.g., 90% absorbent material-free, 95% absorbent material- free, or 99% absorbent material-free, or completely absorbent material-free). A channel may extend through one or more absorbent material layers. The channel generally has a lower bending modulus than the surrounding regions of the absorbent material layer, enabling the material layer to bend more easily and/or rapidly distribute more bodily exudates within the channel than in the surrounding areas of the absorbent material layer. Thus, a channel is not merely an indentation in the material layer that does not create a reduced basis weight in the material layer in the area of the channel.

The term "closed-form" means opposing waist regions are joined, as packaged, either permanently or refastenably to form a continuous waist opening and leg openings.

The term "cross direction (CD)" is used herein to refer to a direction that is generally perpendicular to the machine direction.

The term "Decitex" also known as Dtex is a measurement used in the textile industry used for measuring yarns or filaments. 1 Decitex = 1 gram per 10.000 meters. In other words, if 10.000 linear meters of a yarn or filament weighs 500 grams that yarn or filament would have a decitex of 500.

The term "disposable" is used herein to describe absorbent articles that generally are not intended to be laundered or otherwise restored or reused as an absorbent article i.e., they are intended to be discarded after a single use and, preferably, to be recycled, composted or otherwise disposed of in an environmentally compatible manner.

The term "Dtex-to-Spacing-Ratio" is determined by dividing the elastic decitex by the elastic spacing of the plurality of elastics being examined.

The term "Dtex-to-Nonwoven-Basis-Weight-Ratio" is determined by dividing the elastic decitex by the nonwoven basis weight of the one or more nonwoven substrates of the elastomeric laminate disposed on one side (garment facing side or wearer-facing side) of the elastic strand, i.e. the inner or outer elastomeric laminate substrate layers.

The term "disposed" is used to mean that an element(s) is formed (joined and positioned) in a particular place or position as a unitary structure with other elements or as a separate element joined to another element.

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension of the article and when combined with the term "substantially" includes directions within ±45° of the longitudinal direction.

The term "machine direction (MD)" is used herein to refer to the direction of material flow through a process. In addition, relative placement and movement of material can be described as flowing in the machine direction through a process from upstream in the process to downstream in the process.

The term "nonwoven substrate/fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

The term "spunbond fibers (or layer(s) or nonwovens)" refers to fibers formed by extruding molten thermoplastic polymers as filaments or fibers from a plurality of relative fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 $\mu$m or higher. The spinneret can either be a large spinneret having several thousands holes per meter of width or be banks of smaller spinnerets, for example, containing as few as 40 holes.

The term "spunbond-meltblown-spunbond" (SMS) nonwoven fabric as used herein refers to a multi-layer composite sheet comprising a web of meltblown fibers sandwiched between and bonded to two spunbond layers. A SMS nonwoven fabric can be formed in line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternatively, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated to form laminate layers, for example spunbond-meltblown-meltblown-spunbond (SMMS), or spunbond-meltblown (SM) etc.

The term "meltblown fibers" means fibers formed by extruding a molten thermoplastic material through a plurality of fine, usually circular, die capillaries as molten threads or filaments into a high velocity gas stream (e.g. air) which attenuates the filaments of molten thermoplastic material to reduce their diameter, which may be to microfiber diameter. In general, meltblown fibers have an average fiber diameter of up to about 10 microns. After the fibers are formed, the meltblown fibers are carried by the high velocity gas stream and are deposited on a collecting surface to form a web of randomly disbursed meltblown fibers. The term "open-form" means opposing waist regions are not initially joined to form a continuous waist opening and leg openings but comprise a closure means such as a fastening system to join the waist regions to form the waist and leg openings before or during application to a wearer of the article.

The term "Pre-strain" refers to the strain imposed on an elastic or elastomeric material prior to combining it with another element of the elastomeric laminate or the absorbent article. Pre-strain is determined by the following equation Pre-strain = ((extended length of the elastic -relaxed length of the elastic)/relaxed length of the elastic)* 100.

The terms "proximal" and "distal" refer respectively to the location of an element relatively near to or far from the longitudinal or lateral centerline of a structure (e.g. the proximal edge of a longitudinally extending element is located nearer to the longitudinal axis than the distal edge of the same element is located relative to the same longitudinal axis).

As used herein, the " skin-facing" or "bodyside" or " body-facing" or "user facing" side or surface means that surface of the article or component which is intended to be disposed toward or placed adjacent to the body of the wearer during ordinary use, while the "outward", "outward-facing", "garment-facing" or "garment" side or surface is on the opposite side, and is intended to be disposed to face away from the wearer's body during ordinary use. Such outward surface may be arranged to face toward or placed adjacent to the wearer's undergarments when the absorbent article is worn.

The term "substrate" is used herein to describe a material which is primarily two-dimensional (i.e. in an XY plane) and whose thickness (in a Z direction) is relatively small (i.e. 1/10 or less) in comparison to its length (in an X direction) and with (in a Y direction).

Non- limiting examples of substrates include a web, layer or layers of fibrous materials, nonwovens, films and foils such as polymeric or metallic foils. These materials may be used alone or may comprise two or more layers laminated together. As such, a web is a substrate.

"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable.

THE ABSORBENT ARTICLE

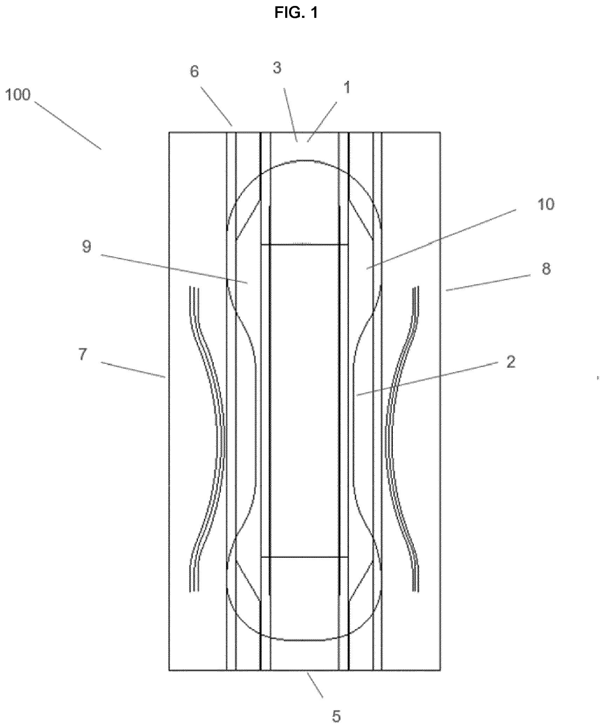

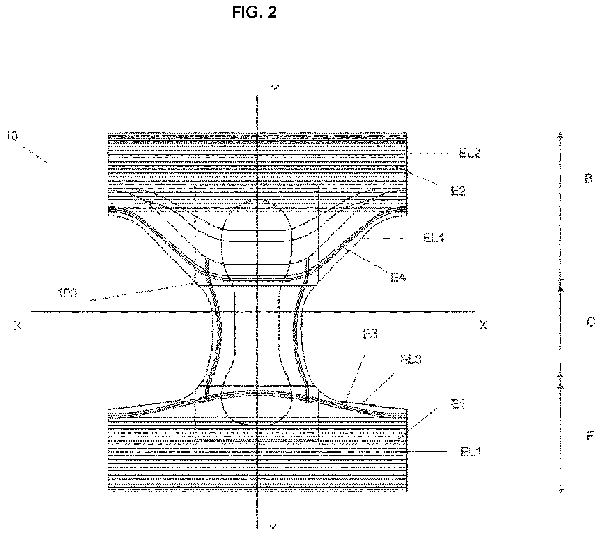

Absorbent articles (10) herein, such as a diaper or a pant-type absorbent article, exemplified in FIG. 2, typically comprise: a liquid permeable topsheet (1); a liquid impermeable backsheet (2); an absorbent core (2) interposed between said topsheet (1) and said backsheet (3), together forming an absorbent article chassis (100) having a front edge (5), a back edge (6) and oppositely disposed side edges (7, 8) connecting said front and back edges (5, 6) and running along a longitudinal axis (y); an elastic-material-comprising component selected from the group consisting of: one or more cuffs (9, 10) extending substantially parallel to said side edges comprising one or more elastic elements, as exemplified in FIG. 1; one or more transversal barriers extending substantially perpendicular to the longitudinal axis (y) and along said front and/or back edges (5, 6), said transversal barriers comprising one or more elastic elements; one or more, preferably at least two, side panels comprising one or more elastic elements, said side panels being joined to said chassis about a proximal end thereof and having a distal end extending away from said chassis (100) along a transverse axis (x) being substantially perpendicular to said longitudinal axis (y); one or more elastic waistbands positioned adjacent said front and/or back edges (103, 104) comprising one or more elastic elements; and combinations thereof.

In an embodiment, the backsheet (3) comprises a liquid-impervious film material. The backsheet may further comprise a backsheet nonwoven material, referred to as the outer cover material.

In a preferred embodiment, an absorbent article (10) comprises a first elastomeric laminate (EL1) forming at least a portion of a belt, a side panel, or an ear panel in a front waist region (F). The first elastomeric laminate (EL1) comprises a first plurality of elastics (E1) disposed between a first substrate layer and a second substrate layer. The first plurality of elastics (E1) comprises an Average-Dtex of less than 600, an Average-Strand-Spacing of more than 4 mm and an Average-Pre-Strain of from 50% to about 400%. Furthermore, the absorbent article (10) comprises a second elastomeric laminate (EL2) forming at least a portion of a belt, a side panel, or an ear panel in a back waist region (B). The second elastomeric laminate (EL2) comprises a second plurality of elastics (E2) between a first substrate layer and a second substrate layer. The second plurality of elastics (E2) comprises an Average-Dtex of less than 600, an Average-Strand-Spacing of more than 4 mm and an Average-Pre-Strain of from 50% to about 400%.

In a preferred embodiment, the first plurality of elastics (E1) and/or the second plurality of elastics (E2) comprise from about 0,1% to about 99,9%wt of recycled polymers by total weight of polymers, preferably from about 1% to about 99%wt of recycled polymers by total weight of polymers; even more preferably from about 2% to about 98%wt of recycled polymers by total weight of polymers; even more preferably from about 3% to about 97%wt of recycled polymers by total weight of polymers; even more preferably from about 4% to about 96%wt of recycled polymers by total weight of polymers; even more preferably from about 5% to about 95%wt of recycled polymers by total weight of polymers; even more preferably from about 6% to about 94%wt of recycled polymers by total weight of polymers; even more preferably from about 7% to about 93%wt of recycled polymers by total weight of polymers; even more preferably from about 8% to about 92%wt of recycled polymers by total weight of polymers; even more preferably from about 9% to about 91 %wt of recycled polymers by total weight of polymers; even more preferably from about 10% to about 90%wt of recycled polymers by total weight of polymers; even more preferably from about 11% to about 89%wt of recycled polymers by total weight of polymers; even more preferably from about 12% to about 88%wt of recycled polymers by total weight of polymers; even more preferably from about 13% to about 87%wt of recycled polymers by total weight of polymers; even more preferably from about 14% to about 86%wt of recycled polymers by total weight of polymers; even more preferably from about 15% to about 85%wt of recycled polymers by total weight of polymers; even more preferably from about 16% to about 84%wt of recycled polymers by total weight of polymers; even more preferably from about 17% to about 83%wt of recycled polymers by total weight of polymers; even more preferably from about 18% to about 82%wt of recycled polymers by total weight of polymers; even more preferably from about 19% to about 81 %wt of recycled polymers by total weight of polymers; even more preferably from about 20% to about 80%wt of recycled polymers by total weight of polymers; even more preferably from about 21% to about 79%wt of recycled polymers by total weight of polymers; even more preferably from about 22% to about 78%wt of recycled polymers by total weight of polymers; even more preferably from about 23% to about 77%wt of recycled polymers by total weight of polymers; even more preferably from about 24% to about 76%wt of recycled polymers by total weight of polymers; even more preferably from about 25% to about 75%wt of recycled polymers by total weight of polymers; even more preferably from about 26% to about 74%wt of recycled polymers by total weight of polymers; even more preferably from about 27% to about 73%wt of recycled polymers by total weight of polymers; even more preferably from about 28% to about 72%wt of recycled polymers by total weight of polymers; even more preferably from about 29% to about 71%wt of recycled polymers by total weight of polymers; even more preferably from about 30% to about 70%wt of recycled polymers by total weight of polymers; even more preferably from about 31 % to about 69%wt of recycled polymers by total weight of polymers; even more preferably from about 32% to about 68%wt of recycled polymers by total weight of polymers; even more preferably from about 33% to about 67%wt of recycled polymers by total weight of polymers; even more preferably from about 34% to about 66%wt of recycled polymers by total weight of polymers; even more preferably from about 35% to about 65%wt of recycled polymers by total weight of polymers; even more preferably from about 36% to about 64%wt of recycled polymers by total weight of polymers; even more preferably from about 37% to about 63%wt of recycled polymers by total weight of polymers; even more preferably from about 38% to about 62%wt of recycled polymers by total weight of polymers; even more preferably from about 39% to about 61%wt of recycled polymers by total weight of polymers; even more preferably from about 40% to about 60%wt of recycled polymers by total weight of polymers; even more preferably from about 41% to about 59%wt of recycled polymers by total weight of polymers; even more preferably from about 42% to about 58%wt of recycled polymers by total weight of polymers; even more preferably from about 43% to about 57%wt of recycled polymers by total weight of polymers; even more preferably from about 44% to about 56%wt of recycled polymers by total weight of polymers; even more preferably from about 45% to about 55%wt of recycled polymers by total weight of polymers; even more preferably from about 46% to about 54%wt of recycled polymers by total weight of polymers; even more preferably from about 47% to about 53%wt of recycled polymers by total weight of polymers; even more preferably from about 48% to about 52%wt of recycled polymers by total weight of polymers; even more preferably from about 49% to about 51%wt of recycled polymers by total weight of polymers; even more preferably about 50%wt of recycled polymers by total weight of polymers. Surprisingly, it has been found that incorporating recycled polymers in the first and/or second plurality of elastics allows for the use of elastics with lower Dtex without negative impact on the performance of the elastics vs standard Dtex value elastics.

In a preferred embodiment, the first plurality of elastics (E1) and/or the second plurality of elastics (E2) comprise from about 0,1% to about 99,9%wt of virgin polymers by total weight of polymers, preferably from about 1% to about 99%wt of virgin polymers by total weight of polymers; even more preferably from about 2% to about 98%wt of virgin polymers by total weight of polymers; even more preferably from about 3% to about 97%wt of virgin polymers by total weight of polymers; even more preferably from about 4% to about 96%wt of virgin polymers by total weight of polymers; even more preferably from about 5% to about 95%wt of virgin polymers by total weight of polymers; even more preferably from about 6% to about 94%wt of virgin polymers by total weight of polymers; even more preferably from about 7% to about 93%wt of virgin polymers by total weight of polymers; even more preferably from about 8% to about 92%wt of virgin polymers by total weight of polymers; even more preferably from about 9% to about 91%wt of virgin polymers by total weight of polymers; even more preferably from about 10% to about 90%wt of virgin polymers by total weight of polymers; even more preferably from about 11% to about 89%wt of virgin polymers by total weight of polymers; even more preferably from about 12% to about 88%wt of virgin polymers by total weight of polymers; even more preferably from about 13% to about 87%wt of virgin polymers by total weight of polymers; even more preferably from about 14% to about 86%wt of virgin polymers by total weight of polymers; even more preferably from about 15% to about 85%wt of virgin polymers by total weight of polymers; even more preferably from about 16% to about 84%wt of virgin polymers by total weight of polymers; even more preferably from about 17% to about 83%wt of virgin polymers by total weight of polymers; even more preferably from about 18% to about 82%wt of virgin polymers by total weight of polymers; even more preferably from about 19% to about 81%wt of virgin polymers by total weight of polymers; even more preferably from about 20% to about 80%wt of virgin polymers by total weight of polymers; even more preferably from about 21% to about 79%wt of virgin polymers by total weight of polymers; even more preferably from about 22% to about 78%wt of virgin polymers by total weight of polymers; even more preferably from about 23% to about 77%wt of virgin polymers by total weight of polymers; even more preferably from about 24% to about 76%wt of virgin polymers by total weight of polymers; even more preferably from about 25% to about 75%wt of virgin polymers by total weight of polymers; even more preferably from about 26% to about 74%wt of virgin polymers by total weight of polymers; even more preferably from about 27% to about 73%wt of virgin polymers by total weight of polymers; even more preferably from about 28% to about 72%wt of virgin polymers by total weight of polymers; even more preferably from about 29% to about 71%wt of virgin polymers by total weight of polymers; even more preferably from about 30% to about 70%wt of virgin polymers by total weight of polymers; even more preferably from about 31% to about 69%wt of virgin polymers by total weight of polymers; even more preferably from about 32% to about 68%wt of virgin polymers by total weight of polymers; even more preferably from about 33% to about 67%wt of virgin polymers by total weight of polymers; even more preferably from about 34% to about 66%wt of virgin polymers by total weight of polymers; even more preferably from about 35% to about 65%wt of virgin polymers by total weight of polymers; even more preferably from about 36% to about 64%wt of virgin polymers by total weight of polymers; even more preferably from about 37% to about 63%wt of virgin polymers by total weight of polymers; even more preferably from about 38% to about 62%wt of virgin polymers by total weight of polymers; even more preferably from about 39% to about 61%wt of virgin polymers by total weight of polymers; even more preferably from about 40% to about 60%wt of virgin polymers by total weight of polymers; even more preferably from about 41 % to about 59%wt of virgin polymers by total weight of polymers; even more preferably from about 42% to about 58%wt of virgin polymers by total weight of polymers; even more preferably from about 43% to about 57%wt of virgin polymers by total weight of polymers; even more preferably from about 44% to about 56%wt of virgin polymers by total weight of polymers; even more preferably from about 45% to about 55%wt of virgin polymers by total weight of polymers; even more preferably from about 46% to about 54%wt of virgin polymers by total weight of polymers; even more preferably from about 47% to about 53%wt of virgin polymers by total weight of polymers; even more preferably from about 48% to about 52%wt of virgin polymers by total weight of polymers; even more preferably from about 49% to about 51%wt of virgin polymers by total weight of polymers; even more preferably about 50%wt of virgin polymers by total weight of polymers. Advantageously, incorporating virgin polymers in the elastic laminates of the present allows for the use of elastics with lower Dtex without negative impact on the performance of the elastics vs standard Dtex value elastics.

In an embodiment, one or both of the first plurality of elastics (E1) and the second plurality of elastics (E2) has an Average-Strand-Spacing of from about 5 mm to about 10 mm, preferably from about 6 mm to about 9 mm, even more preferably from about 7 mm to about 8,5 mm, even more preferably from about 8 mm to about 8,5 mm. Advantageously, these ranges provide for an optimal balance between efficient raw material usage and wearing comfort. A Strand-Spacing that is too high, will increase the pressure that one individual elastic exerts on the skin while a Strand-Spacing that is too low, will increase the amount of raw materials used in the product.

In an embodiment, the absorbent article (10) comprises a first plurality of elastics (E1) and a second plurality of elastics (E2), wherein the Strand-Spacing of the first plurality of elastics (E1) is substantially the same and/or wherein the Strand-Spacing of the second plurality of elastics (E2) is substantially the same. Advantageously, application of elastics within an elastomeric laminate with substantially the same spacing is easier to process on a manufacturing machine.

In an embodiment, the absorbent article (10) comprises a first plurality of elastics (E1) and a second plurality of elastics (E2) wherein the Strand-Spacing of the first plurality of elastics (E1) and the second plurality of elastics (E2) is substantially the same. Advantageously, applying elastics within an elastomeric laminate with substantially the same spacing is easier to process on a manufacturing machine.

In an embodiment, the Average-Strand-Spacing of the first plurality of elastics (E1) and the second plurality of elastics (E2) is substantially the same. Advantageously, applying elastics within an elastomeric laminate with substantially the same spacing is easier to process on a manufacturing machine.

In an embodiment, the first plurality of elastics (E1) and/or the second plurality of elastics (E2) comprises an Average-Dtex from about 100 to about 600 Dtex, more preferably from about 150 to about 600 Dtex, even more preferably from about 200 to about 600 Dtex, even more preferably from about 250 to about 600 Dtex, even more preferably from about 300 to about 600 Dtex, even more preferably from about 350 to about 600 Dtex, even more preferably from about 400 to about 600 Dtex, even more preferably from about 450 to about 600 Dtex, even more preferably from about 500 to about 600 Dtex, and most preferred from about 520 to about 580 Dtex. Advantageously, such ranges of Dtex provide for adequate force and elasticity to assure comfort and fit without the necessity of high Dtex elastics which saves on material cost and has a positive impact on sustainability goals.

In an embodiment, the first and/or second elastomeric laminate (EL1, EL2) has an Average-Dtex-to-Nonwoven-Basis-Weight ratio from about 10 to about 100, more preferably from about 15 to about 95, even more preferably from about 20 to about 90, even more preferably from about 25 to about 85, even more preferably from about 30 to about 80, even more preferably from about 35 to about 75, even more preferably from about 40 to about 70, even more preferably from about 45 to about 65, even more preferably from about 50 to about 60. Advantageously, such ranges of Dtex-to-Nonwoven-Basis-Weight ratio ensure enough strength within the elastomeric laminate by having enough total nonwoven basis weight in combination with having the lowest possible Dtex at a Average-Strand-Spacing of 4 mm or more.

In an embodiment, the first and/or second elastomeric laminate (EL1, EL2) has an Average-Dtex-to-Strand-Spacing ratio from about 20 to about 150, preferably from about 25 to about 145, even more preferably from about 30 to about 140, even more preferably from about 35 to about 135, even more preferably from about 40 to about 130, even more preferably from about 45 to about 125, even more preferably from about 50 to about 120, even more preferably from about 55 to about 115, even more preferably from about 60 to about 110, even more preferably from about 65 to about 105, even more preferably from about 65 to about 100, even more preferably from about 65 to about 95, even more preferably from about 65 to about 90, even more preferably from about 65 to about 85, even more preferably from about 65 to about 80, even more preferably from about 65 to about 75, even more preferably from about 65 to about 70. As the Decitex of elastics increases, the force to extend the elastic also increases. To maintain the proper balance of forces the spacing between the elastics may also be increased. As the decitex decreases, the elastic spacing should also decrease to ensure the proper balance of forces. Advantageously, the elastomeric laminates of the current invention do not require a decrease in spacing by reducing the Dtex of the elastic, because of the use of recycled polymers.

In a preferred embodiment, an absorbent article (10) comprises a third elastomeric laminate (EL3) forming at least a portion of a belt, a side panel, or an ear panel in a front waist region (F). The third elastomeric laminate (EL3) comprises a third plurality of elastics (E3) disposed between a first substrate layer and a second substrate layer. The third plurality of elastics (E3) comprises an Average-Dtex of less than 600, an Average-Strand-Spacing of more than 4 mm and an Average-Pre-Strain of from 50% to about 400%. Furthermore, the absorbent article (10) comprises a fourth elastomeric laminate (EL4) forming at least a portion of a belt, a side panel, or an ear panel in a back waist region (B). The fourth elastomeric laminate (EL4) comprises a fourth plurality of elastics (E4) between a first substrate layer and a second substrate layer. The fourth plurality of elastics (E4) comprises an Average-Dtex of less than 600, an Average-Strand-Spacing of more than 4 mm and an Average-Pre-Strain of from 50% to about 400%. Preferably, the third plurality of elastics (E3) and/or the fourth plurality of elastics (E4) extends in parallel in a curved manner.

In a preferred embodiment, the third plurality of elastics (E3) and/or the fourth plurality of elastics (E4) is positioned closer to the lateral central axis (X-X) than the first plurality of elastics and/or second plurality of elastics. Preferably, the third plurality of elastics (E3) and/or the fourth plurality of elastics (E4) is positioned adjacent the front waist peripheral leg edge and/or the back waist peripheral leg edge.

In a preferred embodiment, the third plurality of elastics (E3) and/or the fourth plurality of elastics (E4) comprises from about 0,1% to about 99,9%wt of recycled polymers by total weight of polymers, preferably from about 1% to about 99%wt of recycled polymers by total weight of polymers; even more preferably from about 2% to about 98%wt of recycled polymers by total weight of polymers; even more preferably from about 3% to about 97%wt of recycled polymers by total weight of polymers; even more preferably from about 4% to about 96%wt of recycled polymers by total weight of polymers; even more preferably from about 5% to about 95%wt of recycled polymers by total weight of polymers; even more preferably from about 6% to about 94%wt of recycled polymers by total weight of polymers; even more preferably from about 7% to about 93%wt of recycled polymers by total weight of polymers; even more preferably from about 8% to about 92%wt of recycled polymers by total weight of polymers; even more preferably from about 9% to about 91%wt of recycled polymers by total weight of polymers; even more preferably from about 10% to about 90%wt of recycled polymers by total weight of polymers; even more preferably from about 11% to about 89%wt of recycled polymers by total weight of polymers; even more preferably from about 12% to about 88%wt of recycled polymers by total weight of polymers; even more preferably from about 13% to about 87%wt of recycled polymers by total weight of polymers; even more preferably from about 14% to about 86%wt of recycled polymers by total weight of polymers; even more preferably from about 15% to about 85%wt of recycled polymers by total weight of polymers; even more preferably from about 16% to about 84%wt of recycled polymers by total weight of polymers; even more preferably from about 17% to about 83%wt of recycled polymers by total weight of polymers; even more preferably from about 18% to about 82%wt of recycled polymers by total weight of polymers; even more preferably from about 19% to about 81%wt of recycled polymers by total weight of polymers; even more preferably from about 20% to about 80%wt of recycled polymers by total weight of polymers; even more preferably from about 21% to about 79%wt of recycled polymers by total weight of polymers; even more preferably from about 22% to about 78%wt of recycled polymers by total weight of polymers; even more preferably from about 23% to about 77%wt of recycled polymers by total weight of polymers; even more preferably from about 24% to about 76%wt of recycled polymers by total weight of polymers; even more preferably from about 25% to about 75%wt of recycled polymers by total weight of polymers; even more preferably from about 26% to about 74%wt of recycled polymers by total weight of polymers; even more preferably from about 27% to about 73%wt of recycled polymers by total weight of polymers; even more preferably from about 28% to about 72%wt of recycled polymers by total weight of polymers; even more preferably from about 29% to about 71%wt of recycled polymers by total weight of polymers; even more preferably from about 30% to about 70%wt of recycled polymers by total weight of polymers; even more preferably from about 31% to about 69%wt of recycled polymers by total weight of polymers; even more preferably from about 32% to about 68%wt of recycled polymers by total weight of polymers; even more preferably from about 33% to about 67%wt of recycled polymers by total weight of polymers; even more preferably from about 34% to about 66%wt of recycled polymers by total weight of polymers; even more preferably from about 35% to about 65%wt of recycled polymers by total weight of polymers; even more preferably from about 36% to about 64%wt of recycled polymers by total weight of polymers; even more preferably from about 37% to about 63%wt of recycled polymers by total weight of polymers; even more preferably from about 38% to about 62%wt of recycled polymers by total weight of polymers; even more preferably from about 39% to about 61%wt of recycled polymers by total weight of polymers; even more preferably from about 40% to about 60%wt of recycled polymers by total weight of polymers; even more preferably from about 41% to about 59%wt of recycled polymers by total weight of polymers; even more preferably from about 42% to about 58%wt of recycled polymers by total weight of polymers; even more preferably from about 43% to about 57%wt of recycled polymers by total weight of polymers; even more preferably from about 44% to about 56%wt of recycled polymers by total weight of polymers; even more preferably from about 45% to about 55%wt of recycled polymers by total weight of polymers; even more preferably from about 46% to about 54%wt of recycled polymers by total weight of polymers; even more preferably from about 47% to about 53%wt of recycled polymers by total weight of polymers; even more preferably from about 48% to about 52%wt of recycled polymers by total weight of polymers; even more preferably from about 49% to about 51%wt of recycled polymers by total weight of polymers; even more preferably about 50%wt of recycled polymers by total weight of polymers. Surprisingly, it has been found that incorporating recycled polymers in the first and/or second plurality of elastics allows for the use of elastics with lower Dtex without negative impact on the performance of the elastics vs standard Dtex value elastics.

In a preferred embodiment, the third plurality of elastics (E3) and/or the fourth plurality of elastics (E4) comprise from about 0,1% to about 99,9%wt of virgin polymers by total weight of polymers, preferably from about 1% to about 99%wt of virgin polymers by total weight of polymers; even more preferably from about 2% to about 98%wt of virgin polymers by total weight of polymers; even more preferably from about 3% to about 97%wt of virgin polymers by total weight of polymers; even more preferably from about 4% to about 96%wt of virgin polymers by total weight of polymers; even more preferably from about 5% to about 95%wt of virgin polymers by total weight of polymers; even more preferably from about 6% to about 94%wt of virgin polymers by total weight of polymers; even more preferably from about 7% to about 93%wt of virgin polymers by total weight of polymers; even more preferably from about 8% to about 92%wt of virgin polymers by total weight of polymers; even more preferably from about 9% to about 91%wt of virgin polymers by total weight of polymers; even more preferably from about 10% to about 90%wt of virgin polymers by total weight of polymers; even more preferably from about 11% to about 89%wt of virgin polymers by total weight of polymers; even more preferably from about 12% to about 88%wt of virgin polymers by total weight of polymers; even more preferably from about 13% to about 87%wt of virgin polymers by total weight of polymers; even more preferably from about 14% to about 86%wt of virgin polymers by total weight of polymers; even more preferably from about 15% to about 85%wt of virgin polymers by total weight of polymers;

even more preferably from about 16% to about 84%wt of virgin polymers by total weight of polymers; even more preferably from about 17% to about 83%wt of virgin polymers by total weight of polymers; even more preferably from about 18% to about 82%wt of virgin polymers by total weight of polymers; even more preferably from about 19% to about 81%wt of virgin polymers by total weight of polymers; even more preferably from about 20% to about 80%wt of virgin polymers by total weight of polymers; even more preferably from about 21% to about 79%wt of virgin polymers by total weight of polymers; even more preferably from about 22% to about 78%wt of virgin polymers by total weight of polymers; even more preferably from about 23% to about 77%wt of virgin polymers by total weight of polymers; even more preferably from about 24% to about 76%wt of virgin polymers by total weight of polymers; even more preferably from about 25% to about 75%wt of virgin polymers by total weight of polymers; even more preferably from about 26% to about 74%wt of virgin polymers by total weight of polymers; even more preferably from about 27% to about 73%wt of virgin polymers by total weight of polymers; even more preferably from about 28% to about 72%wt of virgin polymers by total weight of polymers; even more preferably from about 29% to about 71%wt of virgin polymers by total weight of polymers; even more preferably from about 30% to about 70%wt of virgin polymers by total weight of polymers; even more preferably from about 31% to about 69%wt of virgin polymers by total weight of polymers; even more preferably from about 32% to about 68%wt of virgin polymers by total weight of polymers; even more preferably from about 33% to about 67%wt of virgin polymers by total weight of polymers; even more preferably from about 34% to about 66%wt of virgin polymers by total weight of polymers; even more preferably from about 35% to about 65%wt of virgin polymers by total weight of polymers; even more preferably from about 36% to about 64%wt of virgin polymers by total weight of polymers; even more preferably from about 37% to about 63%wt of virgin polymers by total weight of polymers; even more preferably from about 38% to about 62%wt of virgin polymers by total weight of polymers; even more preferably from about 39% to about 61%wt of virgin polymers by total weight of polymers; even more preferably from about 40% to about 60%wt of virgin polymers by total weight of polymers; even more preferably from about 41% to about 59%wt of virgin polymers by total weight of polymers; even more preferably from about 42% to about 58%wt of virgin polymers by total weight of polymers; even more preferably from about 43% to about 57%wt of virgin polymers by total weight of polymers; even more preferably from about 44% to about 56%wt of virgin polymers by total weight of polymers; even more preferably from about 45% to about 55%wt of virgin polymers by total weight of polymers; even more preferably from about 46% to about 54%wt of virgin polymers by total weight of polymers; even more preferably from about 47% to about 53%wt of virgin polymers by total weight of polymers; even more preferably from about 48% to about 52%wt of virgin polymers by total weight of polymers; even more preferably from about 49% to about 51%wt of virgin polymers by total weight of polymers; even more preferably about 50%wt of virgin polymers by total weight of polymers. Advantageously, incorporating virgin polymers in the elastic laminates of the present allows for the use of elastics with lower Dtex without negative impact on the performance of the elastics vs standard Dtex value elastics.

In an embodiment, one or both of the third plurality of elastics (E3) and the fourth plurality of elastics (E4) has an Average-Strand-Spacing of from about 5 mm to about 10 mm, preferably from about 6 mm to about 9 mm, even more preferably from about 7 mm to about 8,5 mm, even more preferably from about 8 mm to about 8,5 mm. Advantageously, these ranges provide for an optimal balance between efficient raw material usage and wearing comfort. A Strand-Spacing that is too high, will increase the pressure that one individual elastic exerts on the skin while a Strand-Spacing that is too low, will increase the amount of raw materials used in the product.

In an embodiment, the absorbent article (10) comprises a third plurality of elastics (E3) and a fourth plurality of elastics (E4), wherein the Strand-Spacing of the third plurality of elastics (E3) is substantially the same and/or wherein the Strand-Spacing of the fourth plurality of elastics (E4) is substantially the same. Advantageously, application of elastics within an elastomeric laminate with substantially the same spacing is easier to process on a manufacturing machine.

In an embodiment, the absorbent article (10) comprises a third plurality of elastics (E3) and a fourth plurality of elastics (E4) wherein the Strand-Spacing of the third plurality of elastics (E3) and the fourth plurality of elastics (E4) is substantially the same. Advantageously, application of elastics within an elastomeric laminate with substantially the same spacing is easier to process on a manufacturing machine.

In an embodiment, the Average-Strand-Spacing of the third plurality of elastics (E3) and the fourth plurality of elastics (E4) is substantially the same. Advantageously, application of elastics within an elastomeric laminate with substantially the same spacing is easier to process on a manufacturing machine.

In an embodiment, the third plurality of elastics (E3) and/or the fourth plurality of elastics (E4) comprise an Average-Dtex from about 100 to about 600 Dtex, more preferably from about 150 to about 600 Dtex, even more preferably from about 200 to about 600 Dtex, even more preferably from about 250 to about 600 Dtex, even more preferably from about 300 to about 600 Dtex, even more preferably from about 350 to about 600 Dtex, even more preferably from about 400 to about 600 Dtex, even more preferably from about 450 to about 600 Dtex, even more preferably from about 500 to about 600 Dtex, and most preferred from about 520 to about 580 Dtex. Advantageously, such ranges of Dtex provide for adequate force and elasticity to assure comfort and fit without the necessity of high Dtex elastics which saves on material cost and has a positive impact on sustainability goals.

In an embodiment, the third and/or fourth elastomeric laminate (EL3, EL4) has an Average-Dtex-to-Nonwoven-Basis-Weight ratio from about 10 to about 100, more preferably from about 15 to about 95, even more preferably from about 20 to about 90, even more preferably from about 25 to about 85, even more preferably from about 30 to about 80, even more preferably from about 35 to about 75, even more preferably from about 40 to about 70, even more preferably from about 45 to about 65, even more preferably from about 50 to about 60. Advantageously, such ranges of Dtex-to-Nonwoven-Basis-Weight ratio ensure enough strength within the elastomeric laminate by having enough total nonwoven basis weight in combination with having the lowest possible Dtex at an Average-Strand-Spacing of 4 mm or more.

In an embodiment, the third and/or fourth elastomeric laminate (EL3, EL4) has an Average-Dtex-to-Strand-Spacing ratio from about 20 to about 150, preferably from about 25 to about 145, even more preferably from about 30 to about 140, even more preferably from about 35 to about 135, even more preferably from about 40 to about 130, even more preferably from about 45 to about 125, even more preferably from about 50 to about 120, even more preferably from about 55 to about 115, even more preferably from about 60 to about 110, even more preferably from about 65 to about 105, even more preferably from about 65 to about 100, even more preferably from about 65 to about 95, even more preferably from about 65 to about 90, even more preferably from about 65 to about 85, even more preferably from about 65 to about 80, even more preferably from about 65 to about 75, even more preferably from about 65 to about 70. As the Decitex of elastics increases, the force to extend the elastic also increases. To maintain the proper balance of forces the spacing between the elastics may also be increased. As the decitex decreases, the elastic spacing should also decrease to ensure the proper balance of forces. Advantageously, the elastomeric laminates of the current invention do not require a decrease in spacing by reducing the Dtex of the elastic, because of the use of recycled polymers.

In a preferred embodiment, the first and second substrate layer of each of the elastomeric laminates (EL1, EL2, EL3, EL4) each have a basis weight from about 6 gsm to about 30 gsm.

In an embodiment, the absorbent article (10) comprising a front belt and a rear belt wherein the front and rear belts are discrete and joined to a garment-facing surface of the chassis or wherein the front and back belts are integral with the chassis. The front and back belts can be integral with the chassis by having a common substrate, for instance an outer cover layer of the backsheet of the absorbent chassis forming the outer layer and/or inner layer of the front and back belts.

ARRAY OF PACKAGES

In an another aspect of the present invention, an array of packages comprises two or more different sizes of absorbent articles according to the present invention. The array comprises a first package comprising a first absorbent article having a first size and a second package comprising a second absorbent article having a second size, the second size being larger than the first size.

In a preferred embodiment, the array of packages comprises an Array Average-Dtex-to-Nonwoven-Basis-Weight ratio from about 10 to about 100, more preferably from about 15 to about 95, even more preferably from about 20 to about 90, even more preferably from about 25 to about 85, even more preferably from about 30 to about 80, even more preferably from about 35 to about 75, even more preferably from about 40 to about 70, even more preferably from about 45 to about 65, even more preferably from about 50 to about 60.

In an embodiment, the array of packages has an Array Average-Dtex-to-Strand-Spacing ratio from about 20 to about 150, preferably from about 25 to about 145, even more preferably from about 30 to about 140, even more preferably from about 35 to about 135, even more preferably from about 40 to about 130, even more preferably from about 45 to about 125, even more preferably from about 50 to about 120, even more preferably from about 55 to about 115, even more preferably from about 60 to about 110, even more preferably from about 65 to about 105, even more preferably from about 65 to about 100, even more preferably from about 65 to about 95, even more preferably from about 65 to about 90, even more preferably from about 65 to about 85, even more preferably from about 65 to about 80, even more preferably from about 65 to about 75, even more preferably from about 65 to about 70.

In an embodiment, the array of packages comprising a first package and a second package wherein both packages comprise the same brand name and wherein both packages comprise the same sub-brand name.

PACKAGING

In an embodiment, a package, comprising absorbent articles according to the present invention, comprises a paper material having a basis weight less than 100 gsm, more preferably from about 25 gsm to about 90 gsm, even more preferably from about 35 gsm to about 80 gsm, most preferably from about 45 gsm to about 70 gsm. The basis weight of the paper material is measured according to the ISO 536 standard. Advantageously, absorbent articles according to the present invention allow for the use of low basis weight paper packages without increasing the risk of tearing. Paper packaging materials can handle less compression than conventional PE film bags. So, in order to properly fill the paper bag (occupy the entire available volume), some bulk in the front and back belts of absorbent articles, which generally is found undesirable in conventional film bags, has surprisingly been found desirable in paper bags. The absorbent articles of the present invention provide for this bulk together with an increased efficiency of raw material usage by a reduced Dtex of elastics and a more sustainable approach by incorporating recycled polymers in the elastics and by using low basis weight paper material.

ELASTICS

Suitable materials for use in the elastic laminates of the present invention are the Lycra® Ecomade fibers and the Lycra® T400® Ecomade fibers from The LYCRA Company. Both fibers are made with recycled resources in order to reduce waste. The Lycra® Ecomade fibers are partly made with pre-consumer spandex manufacturing waste which his blended with virgin polymer at specific concentrations to keep the same performance as the original Lycra® fiber. The Lycra® T400® Ecomade fiber is made from 50% of recycled PET bottles and

In a preferred embodiment Elastics used in the elastomeric laminates according to the present invention are the T868 LYCRA EnviroFit™ fibers of The LYCRA Company.

Advantageously, using such elastics enables a reduction in fiber weight while maintaining overall end-product performance. Delivering the same performance with lower decitex elastics can result in improved production economics and a reduction in ecological impact.

Furthermore, elastic laminates according to the present invention ensure that adequate softness is retained by preventing the need to increase number of elastics in the laminates due to the use of conventional lower dtex elastics. This would inevitable lead to a reduced feeling of softness as more elastic content is indirectly contacting the skin of the wearer. By using the elastic laminates of the present invention, such disadvantage is avoided and soft touch to the skin is retained.

TEST METHODS

AVERAGE-STRAND-SPACING

Using a ruler calibrated against a certified NIST ruler and accurate to 0,5 mm, measure the distance between the two distal strands within a section to the nearest 0,5 mm, and then divide by the number of strands in that section - 1.

Average-Strand-Spacing = d / (n-1) where n > 1

Report to the nearest 0,1 mm

AVERAGE DECITEX (AVERAGE-DTEX)

The Average Decitex Method is used to calculate the Average-Dtex on a length-weighted basis for elastic fibers present in an entire article, or in a specimen of interest extracted from an article. The decitex value is the mass in grams of a fiber present in 10,000 meters of that material in the relaxed state. The decitex value of elastic fibers or elastic laminates containing elastic fibers is often reported by manufacturers as part of a specification for an elastic fiber or an elastic laminate including elastic fibers. The Average-Dtex is to be calculated from these specifications if available. Alternatively, if these specified values are not known, the decitex value of an individual elastic fiber is measured by determining the cross-sectional area of a fiber in a relaxed state via a suitable microscopy technique such as scanning electron microscopy (SEM), determining the composition of the fiber via Fourier Transform Infrared (FT-IR) spectroscopy, and then using a literature value for density of the composition to calculate the mass in grams of the fiber present in 10,000 meters of the fiber. The manufacturer-provided or experimentally measured decitex values for the individual elastic fibers removed from an entire article, or specimen extracted from an article, are used in the expression below in which the length-weighted average of decitex value among elastic fibers present is determined.

The lengths of elastic fibers present in an article or specimen extracted from an article is calculated from overall dimensions of and the elastic fiber pre-strain ratio associated with components of the article with these or the specimen, respectively, if known. Alternatively, dimensions and/or elastic fiber pre-strain ratios are not known, an absorbent article or specimen extracted from an absorbent article is disassembled and all elastic fibers are removed. This disassembly can be done, for example, with gentle heating to soften adhesives, with a cryogenic spray (e.g. Quick-Freeze, Miller-Stephenson Company, Danbury, CT), or with an appropriate solvent that will remove adhesive but not swell, alter, or destroy elastic fibers. The length of each elastic fiber in its relaxed state is measured and recorded in millimeters (mm) to the nearest mm.

Calculation of Average-Dtex

For each of the individual elastic fibers $f_i$ of relaxed length $L_i$ and fiber decitex value $d_i$ (obtained either from the manufacturer's specifications or measured experimentally) present in an absorbent article, or specimen extracted from an absorbent article, the Average-Dtex for that absorbent article or specimen extracted from an absorbent article is defined as:

$$\text{Average-Dtex} = \frac{\sum_{i=1}^{n}(L_i \times d_i)}{\sum_{i=1}^{n} L_i}$$

where n is the total number of elastic fibers present in an absorbent article or specimen extracted from an absorbent article. The Average-Dtex is reported to the nearest integer value of decitex (grams per 10 000 m).

If the decitex value of any individual fiber is not known from specifications, it is experimentally determined as described below, and the resulting fiber decitex value(s) are used in the above equation to determine Average-Dtex.

Experimental Determination of Decitex Value for a Fiber

For each of the elastic fibers removed from an absorbent article or specimen extracted from an absorbent article according to the procedure described above, the length of each elastic fiber $L_k$ in its relaxed state is measured and recorded in millimeters (mm) to the nearest mm. Each elastic fiber is analyzed via FT-IR spectroscopy to determine its composition, and its density $\rho_k$ is determined from available literature values. Finally, each fiber is analyzed via SEM. The fiber is cut in three approximately equal locations perpendicularly along its length with a sharp blade to create a clean cross-section for SEM analysis. Three fiber segments with these cross sections exposed are mounted on an SEM sample holder in a relaxed state, sputter coated with gold, introduced into an SEM for analysis, and imaged at a resolution sufficient to clearly elucidate fiber cross sections. Fiber cross sections are oriented as perpendicular as possible to the detector to minimize any oblique distortion in the measured cross sections. Fiber cross sections may vary in shape, and some fibers may consist of a plurality of individual filaments. Regardless, the area of each of the three fiber cross sections is determined (for example, using diameters for round fibers, major and minor axes for elliptical fibers, and image analysis for more complicated shapes), and the average of the three areas $\alpha_k$ for the elastic fiber, in units of micrometers squared ($\mu m^2$), is recorded to the nearest 0.1 $\mu m^2$. The decitex $d_k$ of the kth elastic fiber measured is calculated by:

$$0\,m \times a_k \times \rho_k \times 10^{-6}$$

where $d_k$ is in units of grams (per calculated 10,000 meter length), $\alpha_k$ is in units of $\mu m^2$, and $\rho_k$ is in units of grams per cubic centimeter (g/cm³). For any elastic fiber analyzed, the experimentally determined $L_k$ and $d_k$ values are subsequently used in the expression above for Average-Dtex.

AVERAGE-PRE-STRAIN

The Average-Pre-Strain of a specimen are measured on a constant rate of extension tensile tester (a suitable instrument is the MTS Insight using Testworks 4.0 Software, as available from MTS Systems Corp.,

Eden Prairie, MN) using a load cell for which the forces measured are within 1% to 90% of the limit of the cell. Articles are conditioned at 23 °C ± 2 C° and 50% ± 2% relative humidity for 2 hours prior to analysis and then tested under the same environmental conditions.

Program the tensile tester to perform an elongation to break after an initial gage length adjustment. First raise the cross head at 10 mm/min up to a force of 0.05N. Set the current gage to the adjusted gage length. Raise the crosshead at a rate of 100 mm/min until the specimen breaks (force drops 20% after maximum peak force). Return the cross head to its original position. Force and extension data is acquired at a rate of 100 Hz throughout the experiment.

Set the nominal gage length to 40 mm using a calibrated caliper block and zero the crosshead. Insert the specimen into the upper grip such that the middle of the test strip is positioned 20 mm below the grip. The specimen may be folded perpendicular to the pull axis, and placed in the grip to achieve this position. After the grip is closed the excess material can be trimmed. Insert the specimen into the lower grips and close. Once again, the strip can be folded, and then trimmed after the grip is closed. Zero the load cell. The specimen should have a minimal slack but less than 0.05 N of force on the load cell. Start the test program.

From the data construct a Force (N) verses Extension (mm). The Average-Pre-Strain is calculated from the bend in the curve corresponding to the extension at which the nonwovens in the elastic are engaged. Plot two lines, corresponding to the region of the curve before the bend (primarily the elastics), and the region after the bend (primarily the nonwovens). Read the extension at which these two lines intersect, and calculate the % Pre-Strain from the extension and the corrected gage length. Record as % Pre-strain 0.1%. Calculate the arithmetic mean of three replicate samples for each elastomeric laminate and Average-Pre-Strain to the nearest 0.1%.

**Claims**

1. An absorbent article (10) comprising:

   an absorbent chassis (100) comprising a topsheet (1), a backsheet (3) and an absorbent core (2) disposed between the topsheet (1) and the backsheet (3), wherein the absorbent chassis (100) comprises a front end edge (5) and a back end edge (6);
   a first elastomeric laminate (EL1) forming at least a portion of a belt, a side panel, or an ear panel in a front waist region (F); wherein the first elastomeric laminate (EL1) comprises a first plurality of elastics (E1) disposed between a first substrate layer and a second substrate layer and wherein the first plurality of elastics (E1) comprises an Average-Dtex of less than 600, an Average-Strand-Spacing of more than 4 mm and an Average-Pre-Strain of from 50% to about 400%;

a second elastomeric laminate (EL2) forming at least a portion of a belt, a side panel, or an ear panel in a back waist region (B); wherein the second elastomeric laminate (EL2) comprises a second plurality of elastics (E2) between a first substrate layer and a second substrate layer and wherein the second plurality of elastics (E2) comprises an Average-Dtex of less than 600, an Average-Strand-Spacing of more than 4 mm and an Average-Pre-Strain of from 50% to about 400%;

**characterized in that** the first plurality of elastics (E1) and/or the second plurality of elastics (E2) comprise from about 0,1% to about 99,9%wt of recycled polymers by total weight of polymers, preferably from about 10% to about 90%wt of recycled polymers by total weight of polymers.

2. An absorbent article (10) according to claim 1, wherein one or both of the first plurality of elastics (E1) and the second plurality of elastics (E2) has an Average-Strand-Spacing of from about 5 mm to about 10 mm.

3. An absorbent article (10) according to any of the preceding claims, wherein the Strand-Spacing of the first plurality of elastics (E1) is substantially the same; and/or wherein the Strand-Spacing of the second plurality of elastics (E2) is substantially the same.

4. An absorbent article (10) according to claim 3, wherein the Strand-Spacing of the first plurality of elastics (E1) and the second plurality of elastics (E2) is substantially the same.

5. An absorbent article (10) according to any of the preceding claims, wherein the Average-Strand-Spacing of the first plurality of elastics (E1) and the second plurality of elastics (E2) is substantially the same.

6. An absorbent article (10) according to any of the preceding claims, wherein the first plurality of elastics (E1) and/or the second plurality of elastics (E2) comprise an Average-Dtex from about 100 to about 600 Dtex, more preferably from about 150 to about 600 Dtex, even more preferably from about 200 to about 600 Dtex, even more preferably from about 250 to about 600 Dtex, even more preferably from about 300 to about 600 Dtex, even more preferably from about 350 to about 600 Dtex, even more preferably from about 400 to about 600 Dtex, even more preferably from about 450 to about 600 Dtex, even more preferably from about 500 to about 600 Dtex, and most preferred from about 520 to about 580 Dtex.

7. An absorbent article (10) according to any of the preceding claims, wherein the first and/or second elastomeric laminate (EL1, EL2) has an Average-Dtex-to-Nonwoven-Basis-Weight ratio from about 10 to about 100, preferably from about 30 to about 60.

8. An absorbent article (10) according to any of the preceding claims, wherein the first and/or second elastomeric laminate (EL1, EL2) has an Average-Dtex-to-Strand-Spacing ratio from about 20 to about 150, preferably from about 30 to about 90.

9. An absorbent article (10) according to any of the preceding claims, wherein the absorbent article (10) comprises a third elastomeric laminate (EL3) forming at least a portion of a belt, a side panel, or an ear panel comprising a third plurality of elastics (E3) disposed between a first substrate layer and a second substrate layer and a fourth elastomeric laminate (EL4) forming at least a portion of a belt, a side panel, or an ear panel comprising a fourth plurality of elastics (E4) disposed between a first substrate layer and a second substrate layer; wherein the third and fourth elastomeric laminates (EL3, EL4) comprise an Average-Dtex of less than 600, an Average-Strand-Spacing of more than 4 mm and an Average-Pre-Strain of from 50% to about 400%; **characterized in that** the third plurality of elastics (E3) and/or the fourth plurality of elastics (E4) comprise from about 0,1% to about 99,9%wt of recycled polymers by total weight of polymers, preferably from about 10% to about 90%wt of recycled polymers by total weight of polymers; wherein preferably the third plurality of elastics (E3) and/or the fourth plurality of elastics (E4) extends in parallel in a curved manner.

10. An absorbent article (10) according to claim 9, wherein one or both of the third plurality of elastics (E3) and the fourth plurality of elastics (E4) has an Average-Strand-Spacing of from about 5 mm to about 10 mm.

11. An absorbent article (10) according to any of claims 10, wherein the Strand-Spacing of the third plurality of elastics (E3) is substantially the same; and/or wherein the Strand-Spacing of the fourth plurality of elastics (E4) is substantially the same.

12. An absorbent article (10) according to claim 11, wherein the Strand-Spacing of the third plurality of elastics (E3) and the fourth plurality of elastics (E4) is substantially the same.

13. An absorbent article (10) according to any of claims 9-12, wherein the Average-Strand-Spacing of the third plurality of elastics (E3) and the fourth plurality of elastics (E4) is substantially the same.

14. An absorbent article (10) according to any of the claims 9-13, wherein the third plurality of elastics (E3) and/or the fourth plurality of elastics (E4) comprise an Average-Dtex from about 100 to about 600 Dtex, more preferably from about 150 to about 600 Dtex, even more preferably from about 200 to about 600 Dtex, even more preferably from about 250 to about 600 Dtex, even more preferably from about 300 to about 600 Dtex, even more preferably from about 350 to about 600 Dtex, even more preferably from about 400 to about 600 Dtex, even more preferably from about 450 to about 600 Dtex, even more preferably from about 500 to about 600 Dtex, and most preferred from about 520 to about 580 Dtex.

15. An absorbent article (10) according to any of the claims 9-14, wherein the third and/or fourth elastomeric laminate (EL3, EL4) has an Average-Dtex-to-Nonwoven-Basis-Weight ratio from about 10 to about 100, preferably from about 30 to about 60.

16. An absorbent article (10) according to any of the claims 9-15, wherein the third and/or fourth elastomeric laminate (EL3, EL4) has an Average-Dtex-to-Strand-Spacing ratio from about 20 to about 150, preferably from about 30 to about 90.

17. An absorbent article (10) according to any of the preceding claims, wherein the first substrate layer and the second substrate layer of each of the elastomeric laminates (EL1, EL2, EL3, EL4) have each a basis weight from about 6 gsm to about 30 gsm.

18. An absorbent article (10) according to claim 17, wherein the first and second substrate layer of each of the elastomeric laminates (EL1, EL2, EL3, EL4) comprise at least one spunbond layer or wherein at least one of the first and second substrate layer of each of the elastomeric laminates (EL1, EL2, EL3, EL4) comprises at least one meltblown layer.

19. An absorbent article (10) according to any of the preceding claims, wherein the front and back belts are discrete and joined to a garment-facing surface of the chassis; or wherein the front and back belts are integral with the chassis.

20. An absorbent article (10) according to any of the preceding claims, wherein at least one of the pluralities of elastics (E1, E2, E3, E4) comprises curved elastics extending in parallel.

21. A package comprising absorbent articles according to any of the preceding claims, wherein the package comprises a paper material having a basis weight less than 100 gsm.

22. A package according to claim 21, wherein the package comprises a paper material having a basis weight from about 25 gsm to about 90 gsm, more preferably from about 35 gsm to about 80 gsm, and even more preferably from about 45 gsm to about 70 gsm.

23. An array of packages comprising two or more different sizes of absorbent articles, according to any of claims 21-22, the array comprising: a first package comprising a first absorbent article having a first size; a second package comprising a second absorbent article having a second size; wherein the second size is larger than the first size; wherein the array comprises an Array Average-Dtex-to-Nonwoven-Basis-Weight ratio from about 10 to about 100, preferably from about 30 to about 60.

24. An array of packages comprising two or more different sizes of absorbent articles, according to any of the claims 21-23, the array comprising: a first package comprising a first absorbent article having a first size; a second package comprising a second absorbent article having a second size; wherein the second size is larger than the first size; wherein the array comprises an Array Average-Dtex-to-Strand-Spacing ratio from about 20 to about 150, preferably from about 30 to about 90.

FIG. 1

FIG. 2

Europäisches
Patentamt

European
Patent Office

Office européen
des brevets

# EUROPEAN SEARCH REPORT

Application Number

EP 23 21 8904

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/008255 A1 (LAVON GARY D [US] ET AL) 13 January 2022 (2022-01-13) * paragraphs [0131], [0177] – [0188], [0240] – [0245]; claims; examples * ----- | 1-24 | INV. A61F13/49 |
| | | | **TECHNICAL FIELDS SEARCHED (IPC)** |
| | | | A61F |

The present search report has been drawn up for all claims

| Place of search | Date of completion of the search | Examiner |
|---|---|---|
| Munich | 17 April 2024 | Boccignone, Magda |

EPO FORM 1503 03.82 (P04C01)

## EP 4 389 093 A1

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2024

| Patent document cited in search report | Publication date | Patent family member(s) | Publication date |
|---|---|---|---|
| US 2022008255 A1 | 13-01-2022 | NONE | |

For more details about this annex : see Official Journal of the European Patent Office, No. 12/82